# EUROPEAN PATENT SPECIFICATION

(11) **EP 2 038 656 B1**
(45) Date of publication and mention of the grant of the patent: **02.05.2012**
(21) Application number: 07812758.6
(22) Date of filing: 10.07.2007
(51) Int. Cl.: B82Y 15/00, G01N 33/543, G01N 33/58

(54) **ULTRA-SENSITIVE DETECTION OF ANALYTES**
ULTRASENSITIVER NACHWEIS VON ANALYTEN
DETECTION ULTRA-SENSIBLE D'ANALYTES

(30) Priority: 10.07.2006 US 819766 P; 20.12.2006 US 643033
(43) Date of publication of application: 25.03.2009
(73) Proprietor: Nanosphere, Inc., Northbrook, Illinois 60062 (US)
(72) Inventor: MULLER, Uwe, R., Waukegan, IL 60087 (US); LEFEBVRE, Phil, Lincolnshire, IL 60069 (US)
(74) Representative: Grund, Martin
(86) International application number: PCT/US2007/073166
(87) International publication number: WO 2008/008785

(56) References cited:
- WO-A-01/26038
- US-A1- 2002 164 271
- US-A1- 2003 020 910
- US-A1- 2005 112 784
- NAM JWA-MIN ET AL: "Nanoparticle-based bio-bar codes for the ultrasensitive detection of proteins." SCIENCE (NEW YORK, N.Y.) 26 SEP 2003, vol. 301, no. 5641, 26 September 2003 (2003-09-26), pages 1884-1886, XP002476134 ISSN: 1095-9203
- SIIMAN O ET AL: "Immunophenotyping using gold or silver nanoparticle-polystyrene bead conjugates with multiple light scatter." CYTOMETRY 1 DEC 2000, vol. 41, no. 4, 1 December 2000 (2000-12-01), pages 298-307, XP002476172 ISSN: 0196-4763 cited in the application

## Description

### FIELD OF THE INVENTION

The present invention relates to a screening method for detecting for the presence or absence of one or more target analytes, *e.g.*, proteins, nucleic acids, or other compounds in a sample. In one application, the present invention utilizes nucleic acid reporter markers as biochemical barcodes in combination with metallic nanoparticles for detecting differences in plasmon resonance frequency induced by binding of one or more analytes to two or more nanoparticles in a solution with a flow-based (flow cytometry or micro-capillary) method.

### BACKGROUND OF THE INVENTION

The detection of analytes is important for both molecular biology research and medical applications. Diagnostic methods based on fluorescence, mass spectroscopy, gel electrophoresis, laser scanning and electrochemistry are now available for identifying a variety of protein structures. A. Pandey and M. Mann, Nature, 405: 837-846 (2000); S. Fields and O. K. Song, Nature, 340: 245-246 (1989); Ijksma et al., Anal. Chem., 73: 901-907 (2001); and R. F. Service, Science, 287: 2136-2138 (2000). Antibody-based reactions are widely used to identify the genetic protein variants of blood cells, diagnose diseases, localize molecular probes in tissue, and purify molecules or effect separation processes. H. Zole, Monoclonal Antibodies, Springer-Verlag, New York, p.1-5 (2000). For medical diagnostic applications (e.g. malaria and HIV), antibody tests such as the enzyme-linked immunosorbent assay, Western blotting, and indirect fluorescent antibody tests are extremely useful for identifying single target protein structures. J. E. Butler, J. Immunoassay, 21(2 & 3): 165-209 (2000); and Herbrink et al., Tech. Diagn. Pathol., 2: 1-19 (1991). Rapid and simultaneous sample screening for the presence of multiple antibodies would be beneficial in both research and clinical applications. However, it is difficult, expensive, and time-consuming to simultaneously detect several protein structures under assay conditions using the aforementioned related protocols.

Polymerase chain reaction (PCR) and other forms of target amplification have enabled rapid advances in the development of powerful tools for detecting and quantifying DNA targets of interest for research, forensic, and clinical applications. Saiki et al., Science, 230: 1350 (1985); Saiki et al., Science, 239: 487 (1988); Gibbs, R.A., Curr. Opin. Biotechnol., 2: 69 (1991); Bortolin et al., Anal. Chem., 68: 834 (1996); Deiman et al., Mol. Biotechnol., 20: 163 (2002); Stiriba et al, Angew. Chem. Int. Ed., 41: 1329 (2002); Bustin, S.A., Journal of Molecular Endocrinology, 29: 23 (2002). The development of comparable target amplification methods for proteins could dramatically improve medical diagnostics and the developing field of proteomics. MacBeath, G. and Schreiber, S.L., Science 289: 1760 (2000); Zhu et al., Science , 293: 2101 (2001); Haab et al., Genome Biol. 2(2): RESEARCH 0004.1 (2001); Nam et al., J. Am. Chem. Soc., 124, 3820 (2002). Although one cannot yet chemically duplicate protein targets, it is possible to tag such targets with oligonucleotide markers that can be subsequently amplified with PCR and then use DNA detection to identify the target of interest. Sano et al., Science, 258: 120 (1992); Ruzicka et al., Science, 260: 698 (1993); McKie et al., J. Immunol. Methods, 270: 135 (2002); Zhou et al., Nucl. Acids Res., 21: 6038 (1993); Hendrickson et al., Nucl. Acids Res., 23, 522 (1995); Niemeyer et al., Nucl. Acids Res., 27: 4553 (1999); Schweitzer et al., Proc. Natl. Acad. Sci. U.S.A., 97: 10113 (2000); Neimeyer et al., Angew. Chem. Int. Ed., 40: 3169 (2002); Niemeyer, C.M., Trends Biotechnol., 20: 395 (2002). This approach, often referred to as immuno-PCR, allows one to detect proteins with DNA labels in a variety of different formats. To date, all immuno-PCR approaches involve heterogeneous assays, which involve initial immobilization of a target analyte to a surface with subsequent detection using an antibody with a DNA label (for example, see U.S. Patent nos. 5,635,602, and 5,665,539). The DNA label is typically strongly bound to the antibody (either through covalent interactions or streptavidin-biotin binding).

Although these approaches are notable advances in protein detection, they have several drawbacks: 1) limited sensitivity because of a low ratio of DNA identification sequence to detection antibody; 2) slow target binding kinetics due to the heterogeneous nature of the target capture procedure, which increases assay time and decreases assay sensitivity; 3) complex conjugation chemistries that are required to chemically link the antibody and DNA-markers; and 4) require a PCR amplification step. Niemeyer, C.M., Trends Biotechnol., 20: 395 (2002). Therefore, a sensitive, and rapid method for detecting target analytes in a sample that is amenable to multiplexing and easy to implement is needed. For DNA detection methods, many assays have been developed using radioactive labels, molecular fluorophores, chemiluminescence schemes, electrochemical tags, and most recently, nanostructure-based labels. Nicewamer-Pena et al., Science, 294: 137 (2001); Han et al., Nat. Biotechnol. 19: 631 (2001); Zhao et al., J. Am. Chem. Soc. 125: 11474 (2003); Lortie et al., J. Clin. Microbiol. 29: 2250 (1991); Zeph et al., Curr. Microbiol. 22: 79 (1991); Yu et al., J. Am. Chem. Soc., 123: 11155 (2001); Taton et al., Science 289: 1757 (2000); Park et al., Science 295: 1503 (2002); Cao et al., Science, 297: 1536 (2002); Saghatelian et al., J. Am. Chem. Soc. 125, 344 (2003). Although some nanostructure-based methods are approaching PCR in terms of sensitivity, none thus far have achieved the 1-10 copy sensitivity level offered by PCR. A methodology that allows for PCR-like signal amplification without the complexity, expense, and time and labor intensive aspects associated with PCR would provide significant advantages over such PCR-based methods. Methods of synthesizing unique nanoparticle-oligonucleotide conjugates are well known, for example, in U.S. Patent Nos. 6,750,016 and 6,506,564, which are hereby incorporated in their entirety. Previously, a method has been disclosed that utilizes reporter oligonucleotides as biochemical barcodes for detecting one or more analytes in a solution, as described in U.S. Patent Application No. 11/127808, published on February 23, 2006, as U.S. Patent Application Number 2006-0040286-A1, and International Patent Application Nos. PCT/US05/16545, filed May 12, 2005 and PCT/LJS04/20493, filed June 25, 2004. .

Current techniques cover the shift in the frequency of scattered light as a consequence of target-mediated nanoparticle aggregation. Storhoff, J.J, and Lucas, A.D., Nat. Biotechnol 22(7): 883-7 (2004). However, conventional photometric techniques are not sensitive enough to detect low target quantities (e.g. less than attomolar levels) in bulk experiments. Detection of single binding events have been reported using microscopy, by Sonnichsen, C.B., and Reinhard, M., Nat Biotechnol 23(6): 741-5 (2005), but this method is presently hampered by low throughput and is not amenable to automation. The biobarcode assay, such as that disclosed in US Patent Application No 11/127,808, published on February 23, 2006, as U.S. Patent Application Number 2006-0040286-A1, provides high sensitivity but is limited in throughput due to the need for detection of barcodes by hybridization on a slide. Flow cytometry is a means of detecting rare micron-sized cells or particles in large populations, and has become adapted for high-throughput clinical screening (*e.g*. 24-tube and 96-well samplers). Combining the above techniques could offer a new means of rapidly and sensitively detecting barcodes or non-amplified targets via nanoparticle aggregation in a clinically applicable format.

Conventional wisdom in flow cytometry holds that the signal from particles much smaller than one micrometer would be lost in the signal from sample debris and electronic noise and thus remain undetectable. However, the intensity of noble metal nanoparticle plasmon resonance scatter has been reported to be significantly higher than the fluorescence yield from standard fluorophores, suggesting that they should be detectable by flow cytometry. See, for example, the disclosures of Yguerabide, J. and Yguerabide, E.E., Anal Biochem 262(2): 157-76 (1998); Yguerabide, J. and Yguerabide, E.E., Anal Biochem 262(2): 137-56 (1998). Previous work has shown that gold nanoparticles can be used as labels to make cells, (see, for example, Bohmer, R.M. and King, N.J., Cytometry 5(5): 543-6 (1984); Festin, R. and Bjorklund, B., J Immunol Methods 101(1): 23-8 (1987)), or microparticles (see, Siiman, O.K. and Gordon, Cytometry 41(4): 298-307 (2000)), detectable by flow analysis. However, there have been no reports that describe detection of individual nanoparticles by flow cytometry. The preliminary experiments shown below confirm the hypothesis that individual nanoparticles can be detected by this technique, opening new avenues for molecular diagnostics.

A variety of novel bar coding systems have been developed as multiplex testing platforms for applications in biological, chemical and biomedical diagnostics. Instead of identifying a target through capture at a specific locus on an array, target analytes are captured by a bar coded tag, which then uniquely identifies the target akin to putting a UPC barcode on a product. This requires an appropriate surface functionalization to ensure that the correct target is captured with high efficiency. Moreover the tag, or barcode, has to be readable with minimal error and at high speed, typically by flow analysis. For quantitative assays the target may be labeled separately, or the tag may also serve as the label. A great variety of materials and physico-chemical principles have been exploited to generate a plethora of novel bar coding systems. Their advantages compared to microarray based assay platforms include in solution binding kinetics, flexibility in assay design, compatibility with microplate based assay automation, high sample throughput, and with some assay formats, increased sensitivity.

Nam et al (2003, Science, 301, 1884-1886) discloses the detection of a target analyte using nanoparticles wherein a first species of nanoparticles first captures a target analyte, a second species of nanoparticles is used to sandwich the target analytes, said second nanoparticle containing a barcode. After a first capturing step the bar code is released, captured on a chip, and subsequently detected with a third species of nanoparticles on said chip.

The a platform disclosed in US Patent Application 11/127,808, filed May 12, 2005, and published on February 23, 2006, as U.S. Patent Application Number 2006-0040286-A1, uses bar coded nanoparticles for signal amplification, converting a single captured target into a multiplicity of bar codes. For detection and decoding, however, the bar codes have to be first released from the nanoparticle and then recaptured by hybridization on an array and further hybridized with nanoparticle probes for detection. This process adds significant time and reduces the sensitivity of the assay, since thousands of barcodes are required to generate a detectable signal over noise. Moreover, arrays are expensive and the required silver amplification increases assay variability. The invention herein describes a detection technology that avoids these problems.

### SUMMARY OF THE INVENTION

The invention relates to the methods defined in the appended claims.

The invention overcomes many of the problems of the prior art while greatly expanding the flexibility, adaptability and usefulness of techniques directed to the amplification of a signal to facilitate detection. The present invention relates to methods that utilize binding moieties, such as oligonucleotides as biochemical barcodes, for detecting at least one specific target analyte in one solution. The approach takes advantage of recognition elements of specific binding pairs functionalized either directly or indirectly with nanoparticles, and the previous observation that hybridization events that result in the aggregation of gold nanoparticles can significantly alter their physical properties (e.g. optical, electrical, mechanical). Mirkin, et al., Nature, 382: 607-609 (1996); Storhoff et al., Chem. Rev., 99: 1849-1862 (1999); Park et al., Angew. Chem. Int. Ed., 39: 3845-3848 (2000); Taton et al., Science, 289: 1757-1760 (2000); and Park et al., Angew. Chem. Int. Ed., 40: 2909-2912 (2001).

It is well known in the art that metallic nanoparticles will change color when brought into close proximity. This principle has been exploited by functionalizing such nanoparticles with DNA oligonucleotides or with antibodies for the detection of either nucleic acid or protein analytes. The color shift as a function of surface plasmon resonance can be detected most sensitively by observing the frequency of the scattered light. It is further known that fluorescently stained beads, cells or particles can be detected and separated by flow analysis, for example on a flow cytometer or in a microcapillary through laser enhanced fluorescence detection. In this method, particles pass in single file by a detection window, which allows counting of single events (single cells, particles or beads). Due to the confinement of the particles into a small sample volume, isolated from other particles, the background is significantly reduced, enabling signal to background ratios that make single particles detectable. This method can therefore deliver superior sensitivities over other methods, where a signal is measured from a bulk sample. Note that there are many types of coding systems for beads or particles, including shape and size of beads, radio-frequency encoding, or chemical encoding, whereby the signal may be detected by light reflection, diffraction, scatter, or spectral analysis. For example, it is known that metallic nanoparticles can be coded with Raman active dyes that give each particle a unique Raman signature. They are most sensitively detected by surface enhanced Raman spectroscopy (SERS). Methods for incorporating Raman label for target analyte detection can be found in U.S. Application Serial No. 10/172,428, filed June 14, 2002, published November 13, 2003 as U.S. Patent Application Number 20030211488 A1, U.S. Application Serial No. 10/431,341, filed May 7, 2003, published May 6, 2006 as U.S. Patent Application Number 20040086897 A1, and International Application No. PCT/US03/14100, filed May 7, 2003 .

In its current format, the biobarcode technology detects protein and nucleic acid targets through sandwiching between a magnetic bead and an amplifier nanoparticle that is coated with oligonucleotides of specific sequence (barcodes). By releasing the barcodes each captured target is converted into multiple surrogate targets, which are detected via hybridization to a microarray.

In the invention, the array hybridization detection of the above mentioned released barcodes is replaced with a flow based method, such as either a flow cytometer or a microcapillary. Since a minimum of 10,000 target DNA molecules (e.g. barcode molecules) are required in the hybridization solution to generate sufficient hybridization events on a single spot in the microarray to achieve a detectable signal, the flow method is up to 1000 fold more sensitive, since single hybridization events can be measured, and the counting of 10 events may provide sufficient statistical significance.

In another aspect of the present invention, target analytes can be detected directly. Analysis of protein or DNA targets by flow is faster than by capture on a slide or a microplate, as for example in a microplate-based ELISA, because the flow-based analysis affords a homogeneous assay format (*i.e*. the nanoparticle probes that bind target do not have to be separated from nanoparticles that don't bind target), and because in solution hybridization kinetics are much faster than hybridizations to a solid surface. In assays where the presence of only a single target is to be measured, the target can be captured between two metallic nanoparticles, resulting in aggregation of nanoparticles and a change in the extinction characteristics of the nanoparticle probes. The change in the extinction characteristics can be observed Elghanian, R., and Storhoff, J.J., Science 277: 1078-81 (1991) as a color change based on measuring absorbance. Storhoff *et al.* had shown that this can be measured much more sensitively when measuring the scatter light. Storhoff, J.J. and Lucas, A.D., Nat. Biotechnol 22(7): 883-7 (2004). This concept can be exploited via flow analysis by the binding of targets between two nanoparticles. Since each nanoparticle is basically analysed in a small confined volume, it is physically separated from the other particles and therefore even a very small number of aggregates can be detected.

The combination of the biobarcode technology with the flow-based (flow cytometry or microcapillary) barcode detection method brings surprising new advantages over the existing biobarcode technology and conventional flow-based methods. For instance, it is known that more than 10,000 barcodes per assay are required to obtain a detectable signal by hybridization to a slide. Thus, assuming arguendo an amplification of 10 barcodes per captured target, one can detect about 1000 captured targets at best. However, if one could detect, for example, as few as 100 barcodes by flow analysis, the detection limit would improve by two logs.

There are several ways by which the barcodes can be detected in a flow system. For example, a nanoparticle of a particular size, shape, and/or composition can be used as a probe to bind to a barcode specific for a captured target analyte, thereby permitting detection of one type of target analyte in a sample. In another example, aggregation of two nanoparticles having the particular sizes, shapes, and/or compositions (*e.g.* two 30 nm or larger nanoparticles) can be used to bind a specific barcode. Either way, using the present invention the released barcodes do not have to be recaptured on a microarray but can now be detected directly by flow in a simple and homogeneous detection format.

The invention also provides methods for multiplex analysis (*i.e*. detecting multiple target analytes in a sample). In a typical multiplex analysis more than a single target is to be identified in a single assay. In the case of the biobarcode assay, multiple barcodes can be used and decoded. This can be achieved, for example, by binding the released barcodes to either a single or two or more nanoparticles, as described above, whereby each nanoparticle has a unique plasmon resonance frequency due to their specific size, shape, and/or composition. Aggregation of two particles would shift that specific frequency. In certain aspects of the invention, these methods can be combined, whereby some barcodes are detected by the unique signature of single nanoparticles, while others are detected by the unique signatures generated through aggregation of two or more particles. Thus, the unique resonance signatures of the nanoparticles would reveal which barcode is present.

A further method of multiplexing is provided by coding the nanoparticles with Raman active dyes, which can be sensitively detected and decoded in flow by surface enhanced Raman spectroscopy. The main advantage of this type of multiplexing over conventional biobarcode assays, where decoding of barcodes is achieved via hybridization to an array, is assay speed and sensitivity.

Another powerful approach to multiplexing is provided by combining the detectability of single nanoparticles with the coding power of fluorescently labeled microbeads. The microbeads can contain binding moieties as described herein, such that the microbeads can bind to either the target analyte or to the nanoparticles that are bound to the target analyte. Due to the large size of these beads they can be labeled with thousands of fluorescent molecules. The labeled fluorescent molecules further provide for detectability and high coding capacity, achieved by varying the number and type of fluorophors. However, the binding of a single target analyte to such a microbead cannot be detected by conventional fluorescent labels, since that signal is too weak and would furthermore be swamped out by the fluorescence from the microbead.

However, if one of the microbeads now binds a gold nanoparticle via a captured target, then this nanoparticle can be detected by scattered light. The frequencies of fluorescent light and scatter light involved can be chosen not to overlap. It is important to note that the number of photons scattered from a 60-80 nm particle is about 1,000,000 times larger than the number of photons generated by a standard fluorophor label. Thus, a single nanoparticle can be detected, while a "barcoded" microbead would have to bind sufficient target/bead to get labeled with ∼1,000,000 fluorophors. It follows that in order to achieve this much target binding, target molecules have to be in excess of beads in the traditional bead assay, requiring up-front target amplification by PCR. The approach described in this invention would allow for detection of a very small number of targets without amplification, since the binding of a single target to a bead, followed by the binding of a single nanoparticle probe, would make this complex detectable and decodable.

In addition, barcodes or any other target can be captured by magnetic bead or other surface, and can then be labeled with a specific nanoparticle. The labeled target can be removed from the sample matrix, or the sample matrix can be washed away. The labeling particle can be released into solution and counted individually by flow based methods as described herein.

Disclosed is a method for detecting for the presence of one or more target analytes in a sample, wherein the method comprises the steps of:
a) providing a plurality of nanoparticle probes conjugated to binding moieties capable of binding to a first binding site of the target analyte, wherein the nanoparticle probes comprise a metallic material and have an average diameter of less than 200 nanometers;
b) providing a capture surface comprising binding moieties capable of binding to a second binding site of the target analyte;
c) contacting the nanoparticle probes and capture surface with a sample believed to contain target analytes under conditions effective to allow for binding of the target analyte with the nanoparticle probes and the capture surface to form a complex in the presence of the target analyte;
d) optionally washing the capture surface containing the complex formed in (c) to remove all non-bound nanoparticle probes;
e) releasing the captured nanoparticle probes from the capture surface;
f) subjecting the released nanoparticle probes to confinement conditions under which individual nanoparticle probes can be detected;
g) illuminating nanoparticle probes in the confinement conditions with a light beam; and
h) measuring scatter light generated in step (g) to determine the number of released nanoparticle probes as an indicator of the presence of target
analyte in the sample.

Disclosed is a method for detecting for the presence of one or more target analytes in a sample comprising the steps of:
a) providing a first nanoparticle probe conjugated to at least one binding moiety capable of binding to a first binding site of the target analyte, wherein the nanoparticle probes comprise a metallic material and have an average diameter of less than 200 nanometers;
b) providing a second nanoparticle probe conjugated to at least one binding moiety capable of binding to a second binding site of the target analyte, wherein the nanoparticle probes comprise a metallic material and have an average diameter of less than 200 nanometers;
c) contacting the first and second nanoparticle probes with a sample believed to contain target analytes under conditions effective to allow for binding of the target analyte with the binding moieties on the first and second nanoparticle probes to form a complex in the presence of the target analyte;
d) subjecting the sample-nanoparticle probe mixture in (c) to confinement conditions under which individual nanoparticle probes or nanoparticle probe-target complexes can be detected;
e) illuminating the complex with light of a frequency range that covers the plasmon resonance frequency of the nanoparticles; and
f) measuring intensity of scatter light at one or more selected frequencies to differentiate single from complexed nanoparticles,
whereby the presence of complexed particles is indicative of the presence of the target analyte in the sample.

Disclosed is a method for detecting for the presence of one or more target analytes in a sample, wherein the method comprises the steps of:
a) providing a first nanoparticle probe conjugated to at least one binding moiety capable of binding to a first binding site of the target analyte, wherein the nanoparticle probes comprise a metallic material and have an average diameter of less than 200 nanometers;
b) providing a second nanoparticle probe conjugated to at least one binding moiety capable of binding to a second binding site of the target analyte, wherein the nanoparticle probes comprise a metallic material and have an average diameter of less than 200 nanometers;
c) contacting the first and second nanoparticle probes with a sample believed to contain target analytes under conditions effective to allow for binding of the target analyte with the binding moieties on the first and second nanoparticle probes to form a complex in the presence of the target analyte;
d) subjecting the sample-nanoparticle probe mixture in (c) to optical confinement conditions under which individual nanoparticle probes or nanoparticle probe-target complexes can be detected;
e) illuminating the complex with light of a frequency range that covers the plasmon resonance frequency of the nanoparticle probe-target complexes; and
f) measuring the relative particle speed and scatter light intensity to determine size and number of nanoparticle-target complexes, whereby the presence of nanoparticle-target complexes is indicative of the presence of the target analyte in the sample.

In certain aspects, the nanoparticle probes bind to the target analyte indirectly via at least one specific linker molecule.

In other aspects, a binding moiety comprises an oligonucleotide, antibody, aptamer, or some combination thereof.

In another aspects, the binding moieties comprise oligonucleotides, antibodies, aptamers, or some combination thereof.

In certain aspects, the nanoparticle probes are about 10 to about 150 nm in diameter. Representative nanoparticle probes are about 30, 40, 50, 60, or 80 nm in diameter.

In additional aspects, the target analyte is a protein or hapten and the binding moieties are antibodies. The antibodies can be polyclonal antibodies or monoclonal antibodies.

In further aspects, the target analyte is a nucleic acid molecule having a sequence from a genomic DNA or RNA sample and the binding moieties are oligonucleotides, the oligonucleotides having a sequence that is complementary to at least a portion of the genomic sequence. The target nucleic acid can be of eukaryotic, bacterial, fungal or viral origin and can be eukaryotic, bacterial, fungal or viral DNA. Also, the target analyte can be a sequence from episomal DNA sample and the binding moieties are oligonucleotides, the oligonucleotides having a sequence that is complementary to at least a portion of the episomal DNA sequence.

The confinement conditions are generated by flow cytometry or by capillary eletrophoresis, or by other means, whereby the particles can be individually visualized in solution, such as by particle tracking analysis.

In other aspects, at least one of the nanoparticle probes can be further labeled with a Raman active group. The Raman active group can be used in equal or different concentrations with respect to the nanoparticle probe being labeled.

In additional aspects, the nanoparticle probes comprise gold, silver, copper, or platinum. In further aspects, the nanoparticle probes are core-shell nanoparticles.

In yet other aspects, the target analyte can bind to the capture surface indirectly via at least one specific linker molecule.

In other aspects, the capture surface can be a microtiter well. In some aspects, the capture surface containing the complex formed in step (c) above can be isolated or separated from all non-bound nanoparticle probes. In such cases, the capture surface can be a magnetic bead.

In certain aspects, the plurality of nanoparticle probes used in a method of the invention comprises nanoparticle probes of different shapes, each differently shaped nanoparticle probe being conjugated to at least one binding moiety that binds to a different target analyte, and wherein each differently shaped nanoparticle probe creates unique scatter light when illuminated, thereby indicating the presence of the target analyte to which it binds. The plurality of nanoparticle probes can comprise nanoparticle probes of different materials, wherein each nanoparticle probe of different material is conjugated to at least one binding moiety that binds to a different target analyte, and wherein each nanoparticle probe of different material creates unique scatter light when illuminated, thereby indicating the presence of the target analyte to which it binds. The plurality of nanoparticle probes can further comprise nanoparticle probes of different sizes, wherein each nanoparticle probe of different size is conjugated to at least one binding moiety that binds to a different target analyte, and wherein each nanoparticle probe of different size creates unique scatter light when illuminated, thereby indicating the presence of the target analyte to which it binds.

In some aspects, the methods of the invention further comprise a step of providing one or more labeled microbeads that can bind to either the target analyte or to a nanoparticle probe, thereby capable of forming a complex with the nanoparticle probes and the target analyte. In these aspects, at least one microbead can be fluorescently labeled.

### DESCRIPTION OF THE FIGURES

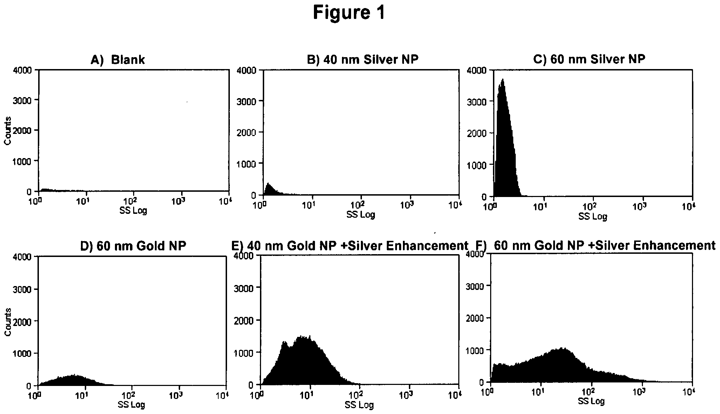

### FIGURE 1- Detection of Gold, Silver, and Silver-coated Gold Nanoparticles By Scatter Using Flow Cytometery

Figure 1 illustrates scatter detection of gold and silver nanoparticles and silver-enhanced gold nanoparticles. Figure 1 shows scatter intensity for a buffer control (no nanoparticles) (Figure 1A); 40 nm silver nanoparticles (1B); 60 nm silver nanoparticles (1C); 60 nm gold nanoparticles (1D); 40 nm gold nanoparticles coated with silver (IE); and 60 nm gold nanoparticles coated with silver (1F), whereby silver staining results in large shifts in side scatter, indicating a significant change in particle size and scatter properties. The Figure supports that the side scatter patterns generated by each type of particle are sufficiently different to allow identification of the nanoparticle's size and composition. This Figure further illustrates that nanoparticles can be detected by flow even when illuminated with a sub-optimal wavelength. The light scatter yield at lambda max is 0.1 for 40 nm Au particles at 535 nm; 0.3 for 60 nm Au particles at 545; 0.55 for 80 nm Au particles at 555 nm; 0.26 for 40 nm Ag particles at 380 nm; 0.55 for 60 nm Ag particles at 380 nm; and 0.88 for 200 nm Ag particles at 610 nm.

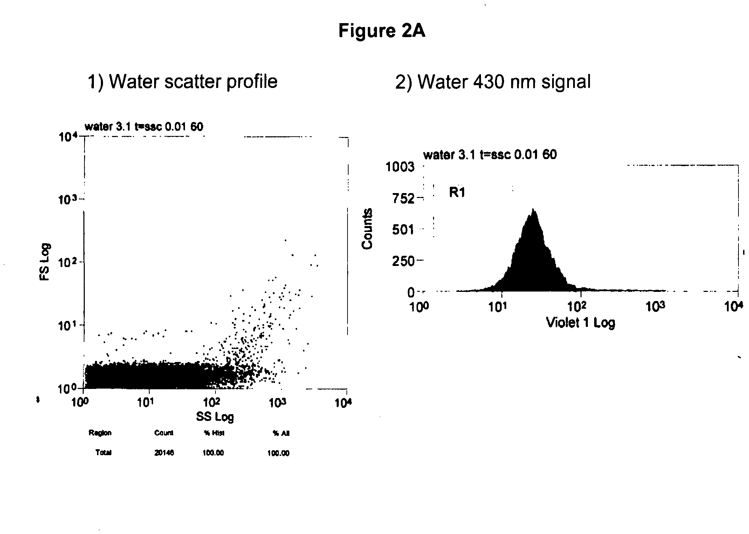

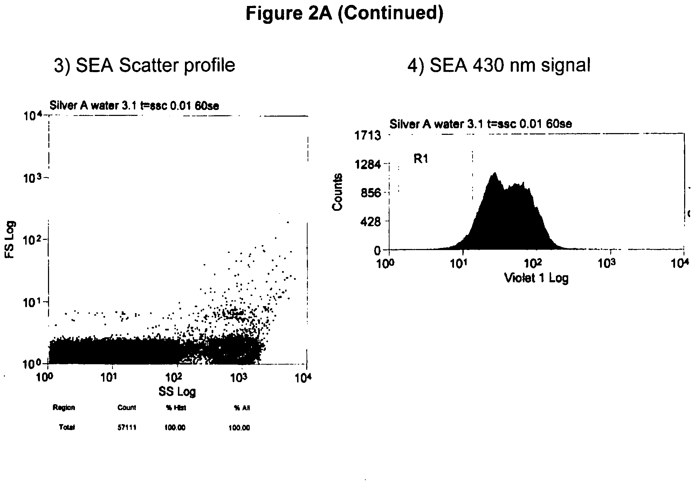

### FIGURE 2A Silver particle detection by plasmon resonance scatter using flow cytometry

Figure 2A shows the detection of silver particles using a 405 nm laser and 430 nm filter (Figure 2A (3)-(4)) compared to water control (Figure 2A (1)-(2)).

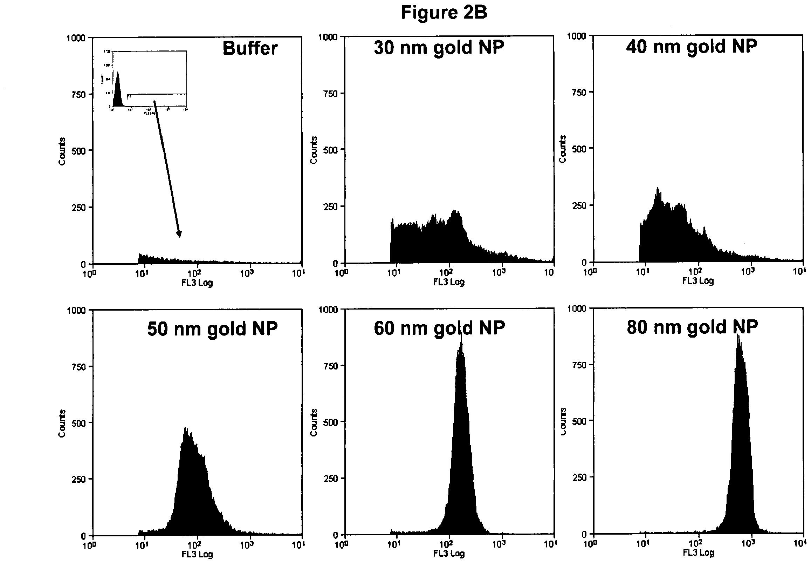

### FIGURE 2B Gold nanoparticle detection by plasmon resonance scatter using flow cytometry

Figure 2B shows detection of different size gold nanoparticles using a 535 nm laser. Similar results were seen when triggering and or detecting with a 535 nm or 565 nm laser.

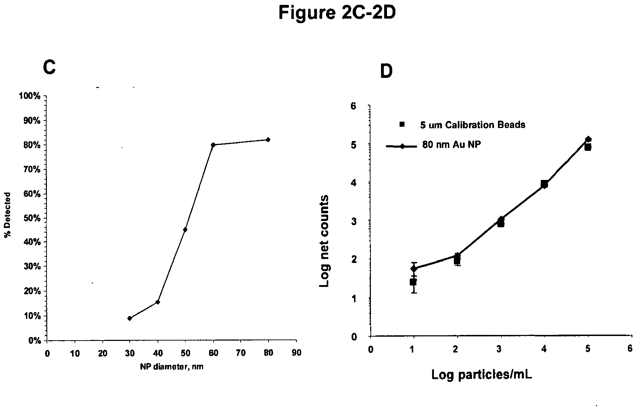

### Figure 2C-2D Efficiency and limit of detection of individual gold nanoparticles

Figure 2C shows the effect of particle diameter on the efficiency of counting gold nanoparticles, and 2D shows that 80 nm particles can be detected with 100% efficiency when counted at 635 nm after triggering the count based on a signal at 535nm. Individual particles are counted even at a concentration as low as 10 particles per ml. For comparison, 5 micrometer diameter calibration beads have been counted.

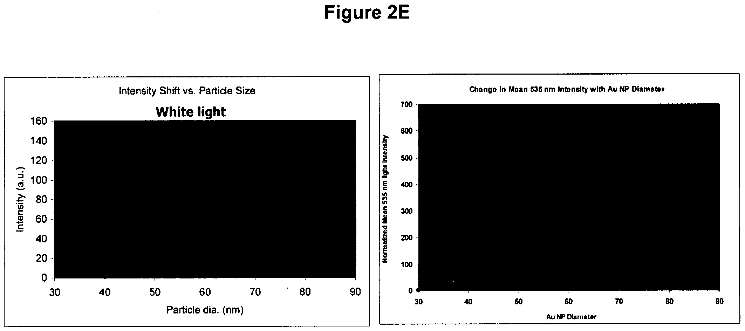

### Figure 2E Intensity changes by 535 nm laser correlate with size, similar to intensity changes induced by white light.

Figure 2E illustrates that intensity changes at 535 nm laser correlate with size, similar to intensity changes induced by white light.

### FIGURE 3 Scatter spectroscopy and sorting of aggregated probes

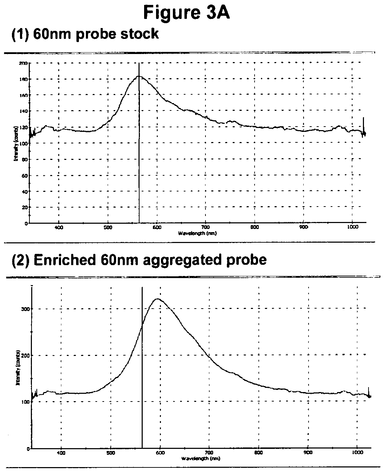

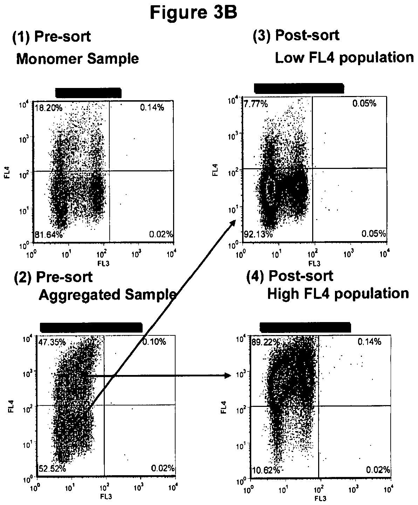

Figure 3 illustrates the correlation of scatter spectroscopy and sorting of aggregated probes. Figure 3A demonstrates results of spectrophotometer white light scatter. Figure 3A shows that the peak wavelength of scattered white light increases when nanoparticles aggregate. Figure 3B demonstrates results of flow cytometer scatter. Figure 3B shows that when analysis of scatter intensity at 635 nm in a flow cytometer is triggered based on a signal at 565 nm, a population of low 635 nm laser intensity counts (FL4-dim) is observed for the monomer sample (1), and an additional population of high intensity counts (FL4-bright) is observed for the aggregated sample (2). The different populations of particles in the aggregated sample were sorted (isolated) on a MoFlo cell sorter based on FL4 intensity. Reanalysis of the 2 sorted populations revealed a sample (3) identical to the monomer sample (1) (low 635 nm laser intensity) for the low FL4 sorted population, and a predominantly high 635 nm intensity population (4) comparable to the FL4-bright population seen in (2). This shows that the changes in peak plasmon resonance wavelength seen when probes are aggregated is due to two different probe populations which can be distinguished by 635 nm laser scatter intensity and physically sorted.

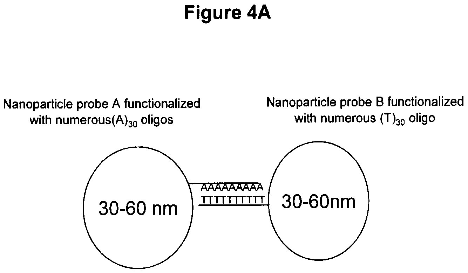

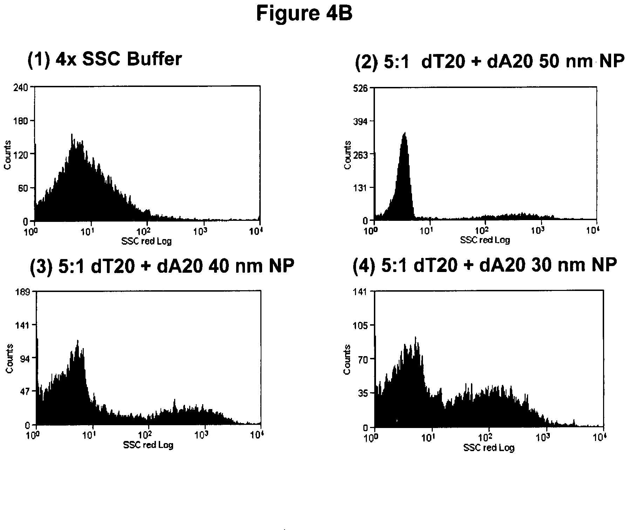

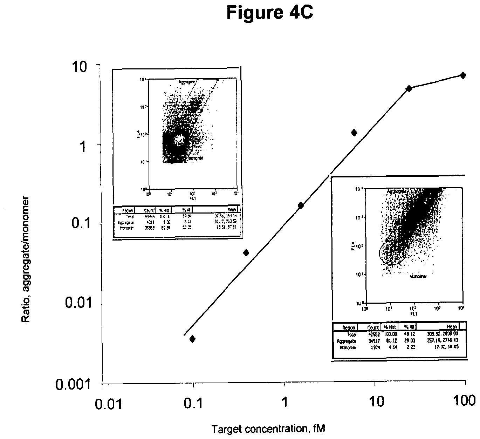

### FIGURE 4 Analysis of rationally aggregated gold nanoparticles

Figure 4A illustrates a nanoparticle probe functionalized with numerous oligonucleotides to allow for hybridizations of monomeric nanoparticles with each other. 30, 40, 50, or 60 nm gold nanoparticles, surface-modified with a multiplicity of either d(A)30 or d(T)30 dithiane androsterone-functionalized oligonucleotides were mixed in 4x saline sodium citrate, (SSC; diluted from 20x stock; Invitrogen). Figure 4B shows the detection of different sized gold nanoparticle monomer probes and those aggregated by DNA hybridization with complementary probes. Change in scatter was detected on the Partec CyFlow ML by triggering on 535 nm signal with the PMT set at 260 V and detecting the 635 nm signal with a PMT setting of 310 (50 nm) to 340 V (30-40 nm); voltages were varied to keep both monomers and aggregates on scale. Figure 4C shows titration and quantification by flow cytometry of 100 fM 60 nm d(A)30 oligonucleotide-functionalized nanoparticle probes with six different concentrations of 60 nm d(T)30 oligonucleotide-functionalized nanoparticle probes in 12 aliquots and assayed using a 96-well plate sampler. The ratio of aggregated probe over monomer probe was plotted vs. d(T)30 probe concentration, revealing a target-induced dose response (Figure 4C), showing changes in aggregated gold nanoparticle concentrations can be quantitatively and reproducibly measured by flow cytometry.

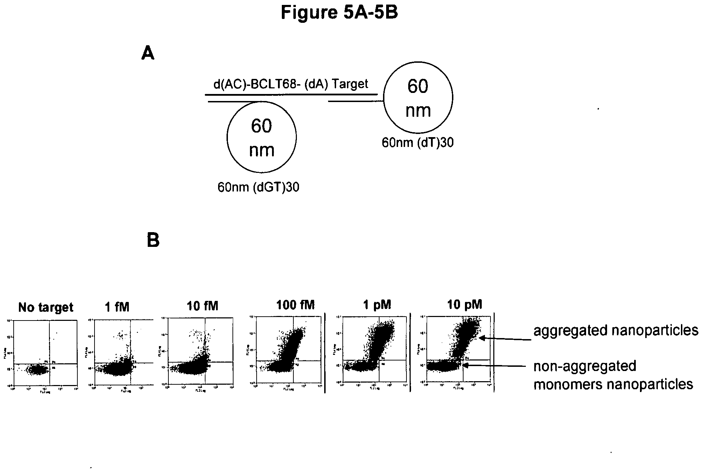

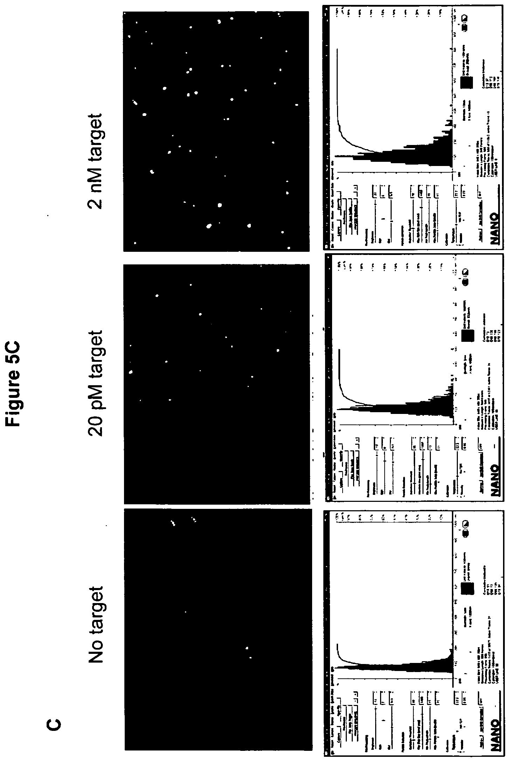

### FIGURE 5 Detection of DNA oligonucleotides hybridized to gold nanoparticle probes by flow analysis

Figure 5 A illustrates a nanoparticle assay format for detecting a target nucleic acid. Each nanoparticle probe has numerous oligos attached thereto, allowing for multiple hybridizations. Figure 5B shows detection of a target oligonucleotide by flow cytometry with different concentrations of target. This experiment also shows that monomers and aggregates can be detected by trigger on 635 nm laser shown as FL4 channel. Aggregated nanoparticles are FL4-bright, and non-aggregated monomers are FL4-dim. Increasing target concentrations increased the amount of aggregated gold NP, represented by an increase in a FL4-bright population and a decrease in the FL4-dim population. Figure 5C shows that nanoparticle aggregation induced by nucleic acid hybridization in a homogeneous assay can be detected under 650 nm laser illumination via microscopy. Figure 5C demonstrates that visualization of nanoparticles using a particle analysis instrument such as a microscope can detect target-induced changes in particle size by visual confinement and analysis of each individual particle in a homogeneous assay (Figure 5C, top panels). The underlying concept is that the scatter signal from individual nanoparticles or nanoparticle aggregates can be compared to their immediate surrounding environment (background), which simulates the physical isolation that is achieved in a flow cytometer; and quantified by the instrument's image analysis software. Aggregated particles become brighter due to enhanced plasmon resonance. The instrument software compares particles to each other by measuring Brownian motion and outputs the data on a histogram that shows increased mean particle size in the presence of increased concentrations of oligonucleotide target (Figure 5C, bottom panels).

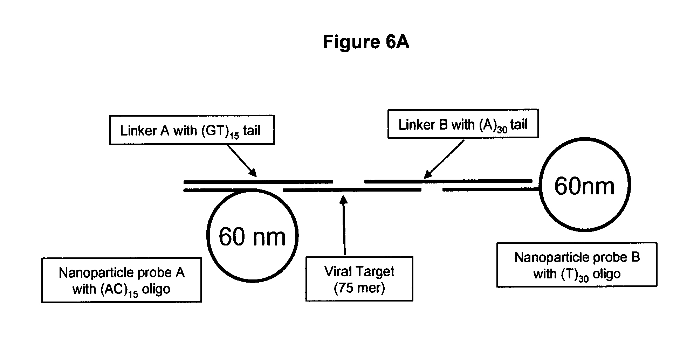

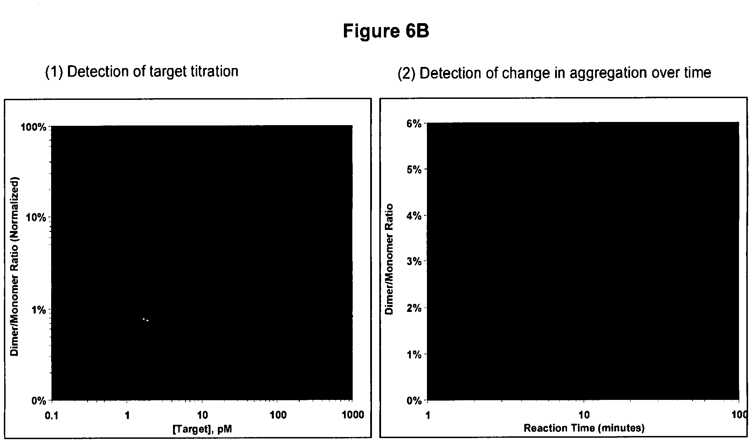

### FIGURE 6 Detection of a viral target analyte having at least a first portion and a second portion (Example 6)

Figure 6A illustrates the detection of a viral target having at least a first portion and a second portion. The detection of the target is accomplished using two linker oligos (linker A and linker B), a nanoparticle probe A, and a nanoparticle probe B. Nanoparticle probe A is conjugated with at least one poly d(AC) oligo. Linker A comprises at least a first portion and a second portion, said first portion comprising a poly d(GT) oligo (complementary to the poly-d(AC) oligo conjugated to Nanoparticle probe A) and said second portion comprising a sequence complementary to the first portion of the viral target. Nanoparticle probe B is conjugated with at least one poly-dT oligos. Linker B comprises a first portion and a second portion, said first portion comprising a poly dA oligo (complementary to the poly dT oligo conjugated to Nanoparticle probe B), and said second portion comprising a sequence complementary to the second portion of the viral target. Each nanoparticle probe would have numerous oligos attached thereto, allowing for multiple hybridizations. Figure 6B shows that (1) increased amounts of the oligo target induced increased amounts of aggregates, and (2) in the presence of constant target and probe, the fraction of aggregates increase over time.

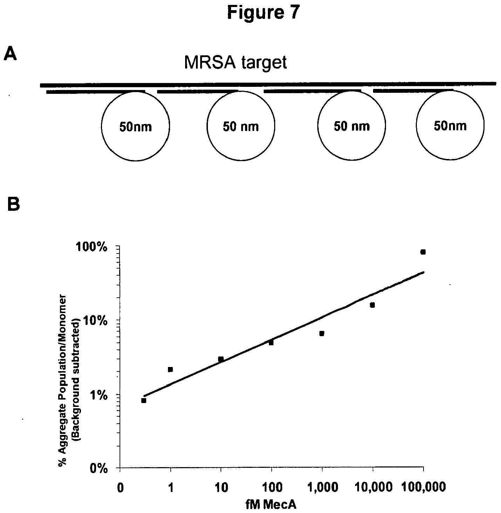

### FIGURE 7 Detection of bacterial target using multiple nanoparticle probes

Figure 7A illustates a nanoparticle assay format for detecting bacterial target. Figure 7A shows a complex of methicillin resistant *Staphylococcus aureus* (MRSA) target with four 50 nm nanoparticle probes, each nanoparticle probe would have numerous oligos attached thereto, allowing for multiple hybridizations. Figure 7B shows the results of a MecA assay to detect MRSA using four 50 nm nanoparticle probes; increased amounts of DNA target induced an increased ratio of FL4-bright population over FL4-dim population at nm 635. A MecA assay was designed to detect Methicillin resistant Staphylococcus aureus (MRSA) by hybridization to four 50 nm nanoparticle probes. 10 pM total nanoparticle probes (2.5 pM of each type) in 10 uL 3.6X SSC/10% Formamide/0.05% Tween 20/2.5% dextran sulfate plus various concentrations of a 281 bp target sequence were mixed and incubated for hybridization. For demonstration purposes the target sequence was derived by an AmpliTaq PCR amplification of the MecA gene of MRSA (strain #700699 obtained from American Type Culture Collection, Manassas, VA) . The hybridization reaction was incubated at room temperature for up to 1.25 hours, then diluted in 4X SSC and subjected to flow analysis. The scatter signal obtained resulting from illumination with a 565 nm laser was used as trigger and the signal intensity obtained by illuminating with a 635 nm signal was used to count nanoparticles and nanoparticle aggregates. A signal specific for dimers and larger aggregates was observed to increase above background with as little as 300 aM of target DNA (200 copies/ul of reaction, 2,000 copies analyzed by flow).

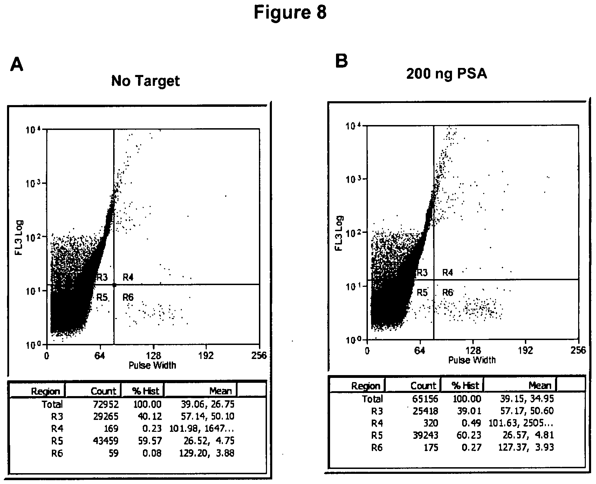

### FIGURE 8 Detection of proteins by nanoparticle aggregation

Figure 8 shows detection of prostate specific antigen (PSA) using 30 nm nanoparticle probes and flow cytometry, demonstrating application of assay to protein target detection.

### DETAILED DESCRIPTION OF THE INVENTION

Unless otherwise required by context, singular terms shall include pluralities and plural terms shall include the singular.

As utilized in accordance with the present disclosure, the following terms, unless otherwise indicated, shall be understood to have the following meanings:

The terms "target," "analyte" or "target analyte" refer to the compound or composition to be detected, including drugs, metabolites, pesticides, pollutants, and the like. The analyte can be comprised of a member of a specific binding pair (sbp) and may be a ligand, which is monovalent (monoepitopic) or polyvalent (polyepitopic), preferably antigenic or haptenic, and is a single compound or plurality of compounds, which share at least one common epitopic or determinant site. The analyte can be a part of a cell such as bacteria or a cell bearing a blood group antigen such as A, B, O, etc., or an HLA antigen or a microorganism, e.g., bacterium, fungus, protozoan, or virus. If the analyte is monoepitopic, the analyte can be further modified, e.g. chemically, to provide one or more additional binding sites. In practicing this invention, the analyte has at least two binding sites. The monoepitopic ligand analytes will generally be from about 100 to 2,000 molecular weight, more usually from 125 to 1,000 molecular weight. Typical analytes may be much larger and include, but are not limited to episomal DNA, genomic DNA, viral nucleic acid molecules, proteins, peptides, nucleic acid segments, molecules, cells, microorganisms and fragments and products thereof, or any substance for which attachment sites, binding members or receptors (such as antibodies) can be developed.

As used herein, the terms "barcode", "biochemical barcode", "biobarcode", "reporter barcode" etc. are all interchangeable with each other and have the same meaning. In the preferred embodiment of the present invention, the biobarcodes are nucleic acids. The markers may be the same, or may be different. The biobarcode assay has been disclosed in U.S. Patent Application 11/127,808, filed May 12, 2005, and published on February 23, 2006, as U.S. Patent Application Number 2006-0040286-A1, U.S. Patent Application 10/877,750, filed June 25, 2004, and published on February 17, 2005, as U.S. Patent Application Number 2005-0037397-A1, International Patent Application PCT/US04/020493 (Publication No. WO05/003394), filed June 25, 2004, and International Patent Application PCT/US05/16545 (Publication No. WO2006/078289), filed May 12, 2005 .

The polyvalent ligand analytes will normally be larger organic compounds, often of polymeric nature, such as polypeptides and proteins, polysaccharides, nucleic acids, and combinations thereof. Such combinations include components of bacteria, viruses, chromosomes, genes, mitochondria, nuclei, cell membranes and the like.

For the most part, the polyepitopic ligand analytes to which the invention can be applied will have a molecular weight of at least about 5,000, more usually at least about 10,000. In the polymeric molecule category, the polymers of interest will generally be from about 5,000 to 5,000,000 molecular weight, more usually from about 20,000 to 1,000,000 molecular weight; among the protein analytes of interest, the molecular weights will usually range from about 5,000 to 200,000 molecular weight.

A wide variety of proteins may be considered as belonging to the family of proteins having similar structural features, proteins having particular biological functions, proteins related to specific microorganisms, particularly disease causing microorganisms, etc. Such proteins include, for example, immunoglobulins, cytokines, enzymes, hormones, cancer antigens, nutritional markers, tissue specific antigens, etc.

The types of proteins, blood clotting factors, protein hormones, antigenic polysaccharides, microorganisms and other pathogens of interest in the present invention are specifically disclosed in U.S. Pat. No. 4,650,770 .

The analyte may be a molecule found directly in a sample, such as a body fluid from a host. The sample can be examined directly or may be pretreated to render the analyte more readily detectible. Furthermore, the analyte of interest may be determined by detecting an agent probative of the analyte of interest such as a specific binding pair member complementary to the analyte of interest, whose presence will be detected only when the analyte of interest is present in a sample. Thus, the agent probative of the analyte becomes the analyte that is detected in an assay. The body fluid can be, for example, urine, blood, plasma, serum, saliva, semen, stool, sputum, cerebral spinal fluid, tears, mucus, and the like.

The term "sample" as used herein refers to any quantity of a substance that may comprise target analytes, and that can be used in a method of the invention. For example, the sample can be a biological sample or can be extracted from a biological sample derived from humans, animals, plants, fungi, yeast, bacteria, viruses, tissue cultures or viral cultures, or a combination of the above. A sample may contain or be extracted from solid tissues (*e.g.* bone marrow, lymph nodes, brain, skin), body fluids (*e.g*. serum, blood, urine, sputum, seminal or lymph fluids), skeletal tissues, or individual cells. Alternatively, the sample can comprise purified or partially purified nucleic acid molecules and, for example, buffers and/or reagents that are used to generate appropriate conditions for successfully performing a method of the invention. In certain embodiments, a sample is or is in solution, and can be subject to flow based detection methods as described herein.

The term "particle" as used herein specifically encompasses both nanoparticles and microparticles as defined and described hereinbelow. As used herein, the term "particle" refers to a small piece of matter that can preferably be composed of metals, silica, silicon-oxide, or polystyrene. A "particle" can be any shape, such as spherical or rod-shaped.

In certain embodiments, the methods of the invention involve the use of nanoparticle probes. Nanoparticles useful in the practice of the invention include metal (e.g., gold, silver, copper and platinum), colloidal materials. The size of the nanoparticles is preferably from about 30 nm to about 200 nm (mean diameter). The nanoparticles can be any shape, such as spherical or rod-shaped. As used herein, a "metallic" nanoparticle comprises at least one metal.

Methods of making metal nanoparticles are well-known in the art. See, e.g., Schmid, G. (ed.) Clusters and Colloids (VCH, Weinheim, 1994); Hayat, M. A. (ed.) Colloidal Gold: Principles, Methods, and Applications (Academic Press, San Diego, 1991); Massart, R., IEEE Taransactions On Magnetics, 17, 1247 (1981); Ahmadi, T. S. et al., Science, 272, 1924 (1996); Henglein, A. et al., J. Phys. Chem., 99, 14129 (1995); Curtis, A. C., et al., Angew. Chem. Int. Ed. Engl., 27, 1530 (1988). See also US Patent No. 6,506,564 .

Nanoparticles useful in the methods of the invention can also be core-shell particles such as the ones described in U.S. Patent application no. 10/034,451, filed December 28, 2002, now U.S. Patent No. 7,238,472 and International application no. PCT/US01/50825, filed December 28, 2002.

Suitable nanoparticles are also commercially available from, e.g., Ted Pella, Inc. (gold and silver), Amersham Corporation (gold), Nanoprobes, Inc. (gold), Nanoparts (gold).

For stability purposes, a nanoparticle probe can have zero, one, or a plurality of oligonucleotides, as well as the binding moieties, attached to it. For example, nanoparticles can be incubated with binding moieties and oligonucleotides in a 3:1 ratio. In one embodiment, the oligonucleotides are polyadenosine oligonucleotides, for example A₁₀, which is an oligonucleotide consisting of 10 adenosines.

Those of skill in the art will appreciate that nanoparticles can be designed to have different scatter light properties based on their size, composition, and shape. Thus, one of skill in the art can select a particular size, composition, and/or shape to represent the presence of a particular target analyte. For example, a gold, round, 30 nm nanoparticle will cause different scatter light than a silver, 60 nm, rod-shaped nanoparticle. Consequently, both probes can be used in one sample to detect the presence of two different target analytes, as discussed herein.

As used herein, the term "linker molecule" refers to a binding moiety that serves as an indirect link between a nanoparticle probe and a target analyte, or between a capture surface and a target analyte. A linker molecule can be a "linker oligonucleotide" with at least two binding regions, one of which binds to a complementary oligonucleotide conjugated to a nanoparticle or capture surface, and the other which binds to a complementary portion of the target analyte. Other examples of linker molecules include streptavidin, avidin, or antibodies. Alternatively, linkers can be generated from any of the binding moieties described below, whereby, for example, two different moieties chemically linked now having specificity for two different binding partners.

The term "binding moiety" is used herein to refer to members of a specific binding pair. The term "specific binding pair (sbp) member" refers to one of two different molecules, which specifically binds to and can be defined as complementary with a particular spatial and/or polar organization of the other molecule. The members of the specific binding pair can be referred to as ligand and receptor (antiligand). These will usually be members of an immunological pair such as antigen-antibody, although other specific binding pairs such as biotin-avidin, enzyme-substrate, enzyme-antagonist, enzyme-agonist, drug-target molecule, hormones-hormone receptors, nucleic acid duplexes, IgG-protein A/protein G, polynucleotide pairs such as DNA-DNA, DNA-RNA, protein-DNA, lipid-DNA, lipid-protein, polysaccharide-lipid, protein-polysaccharide, nucleic acid aptamers and associated target ligands (*e.g*., small organic compounds, nucleic acids, proteins, peptides, viruses, cells, etc.), and the like are not immunological pairs but are included in the invention and the definition of sbp member. A member of a specific binding pair can be the entire molecule, or only a portion of the molecule so long as the member specifically binds to the binding site on the target analyte to form a specific binding pair.

The term "ligand" refers to any organic compound for which a receptor naturally exists or can be prepared. The term ligand also includes ligand analogs, which are modified ligands, usually an organic radical or analyte analog, usually of a molecular weight greater than 100, which can compete with the analogous ligand for a receptor, the modification providing means to join the ligand analog to another molecule. The ligand analog will usually differ from the ligand by more than replacement of a hydrogen with a bond, which links the ligand analog to a hub or label, but need not. The ligand analog can bind to the receptor in a manner similar to the ligand. The analog could be, for example, an antibody directed against the idiotype of an antibody to the ligand.

The term "receptor" or "antiligand" refers to any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, nucleic acid aptamers, avidin, protein A, barstar, complement component C1q, and the like. Avidin is intended to include egg white avidin and biotin binding proteins from other sources, such as streptavidin.

The term "specific binding" refers to the specific recognition of one of two different molecules for the other compared to substantially less recognition of other molecules. Generally, the molecules have areas on their surfaces or in cavities giving rise to specific recognition between the two molecules. Examples of specific binding are antibody-antigen interactions, enzyme-substrate interactions, polynucleotide interactions, and so forth.

The term "non-specific binding" refers to the binding between molecules that is relatively independent of specific surface structures. Non-specific binding may result from several factors including hydrophobic interactions between molecules.

In certain embodiments, a label can be used to further differentiate a target analyte in a sample. For example, nanoparticle probes can serve as labels directly, or their optical properties can be modified by linkage to a Raman-active group. "Raman label," as used herein, is any substance which produces a detectable Raman spectrum, which is distinguishable from the Raman spectra of other components present, when illuminated with a radiation of the proper wavelength. The following is a listing of some, but not all potential candidates for these Raman-active label: 4-(4-Aminophenylazo)phenylarsoni- c acid monosodium salt, arsenazo I, basic fuchsin, Chicago sky blue, direct red 81, disperse orange 3, HABA (2-(4-hydroxyphenylazo)-benzoic acid), erythrosin B, trypan blue, ponceau S, ponceau SS, 1,5-difluoro-2,4-dinitrobenzene, cresyl violet and p-dimethylaminoazobenzene.

A "capture surface" as used herein can be any surface capable of having antibodies, aptamers, oligonucleotides, or analytes bound thereto. Such surfaces include, but are not limited to, glass, metal, plastic, or materials coated with a functional group designed for binding of antibodies, aptamers, oligonucleotides, or analytes. The coating may be thicker than a monomolecular layer; in fact, the coating could involve porous materials of sufficient thickness to generate a porous 3-dimensional structure into which the antibodies, aptamers, oligonucleotides, or analytes can diffuse and bind to the internal surfaces. Binding of antibodies, aptamers, oligonucleotides, or analytes to a substrate can be accomplished by any method known to those of skill in the art and as described, for example, in U.S. Patent Application No. 11/124,609, filed May 6, 2005, published on December 29, 2005, as U.S. Patent Application Number 2005-0287560-A1

A "capture surface" suitable for the methods of the invention include, but are not limited to, microplates, glass slides, nanoparticles, magnetic beads, or any suitable inorganic or organic molecule of sufficient size, or a combination thereof, that offers the appropriate surface for attachment of antibodies, aptamers, oligonucleotides, or analytes, and shows a minimum of non-specific binding to nanoparticle probes that are not bound to target analytes. In one embodiment, the surface is a magnetic (e.g., ferromagnetite) colloidal material. The complex formed between the nanoparticle probe, the target analyte, and the magnetic surface can be easily separated from any unbound components by the application of a magnetic field. In another embodiment, the complex can be separated by centrifugation. In certain embodiments, the magnetic surface is a magnetic bead, such as a magnetic microparticle.

In certain embodiments, a nanoparticle probe bound to target analyte forms a complex with the capture surface through binding of the target analyte to a binding moiety that is attached to the capture surface itself. Once the complex is formed, any unbound probes are removed from the complex by suitable methods, such as, without limitation, washing, centrifugation, and application of a magnetic field. The complex can be disrupted by releasing the nanoparticle from a capture surface using techniques well known to those of skill in the art. For example, specifically bound probes can be selectively released from the capture surface by any suitable methods, including but not limited to, target analyte displacement, epitope displacement, antibody displacement, aptamer displacement, target analyte destruction, antibody destruction, aptamer destruction, protease digestion, restriction digestion, a reducing agent, RNaseH digestion, chemical cleavage, and dehybridization, depending on what binding moiety is used to capture the target analyte bound nanoparticle probes.

In some instances, a "detaching agent" can be used to release the capture nanoparticle probes from the capture surface. As used herein, a "detaching agent" refers to a solution or agent that can disrupt or destruct the linkage of a binding moiety to the capture surface, and detach and release the binding moiety in complex with the nanoparticle probe into solution. For example, where the binding moiety is an oligonucleotide, it can be detached and released from the capture surface by dehybridization, dissolution, or chemical cleavage. Representative detaching agents include, without limitation, iodine, a cyanide salt, and a basic agent. See also U.S. Patent Application No. 11/127808, published on February 23, 2006, as U.S. Patent Application Number 2006-0040286-A1, and International Patent Application Nos. PCT/US05/16545, filed May 12, 2005 and PCT/US04/20493, filed June 25, 2004.

In certain embodiments, a sample having been contacted with nanoparticle probes will be spatially confined in a sample stream under confinement conditions (such as those described in the Examples herein). As used herein, "confinement conditions" refer to the spatial arrangement of the sample in such a manner that allows for analysis of individual nanoparticle probes within the sample either by individual particle tracking in a static solution using particle analysis systems or by spatial manipulations using flow-based methods (*e.g.* flow cytometry or microcapillary electrophoresis). Confinement can be accomplished using methods well known to those of skill in the art. Conventional methods involve "electrokinetic focusing," as discussed, for example, in US Patent No. 6,120,666 . Electrokinetic techniques include electroosmosis and/or electrophoresis. Two common types of electrophoresis are steady state and capillary zone electrophoresis as discussed by Hahm and Beskok, 2005, Bull. Polish Acad. Sci. 53:325-334 (2005). Once in confinement conditions, the sample stream can be illuminated with a light beam. The confinement conditions permit the nanoparticle probes to be isolated against a particle-free background, or to flow single-file past the light beam, such as a laser beam (and in many instruments, past two or more laser beams). The momentary pulse of scatter light emitted as the particle crosses the beam is measured by photomultipliers at some angle (typically 90 degree angle) from the beam. Typically, two to three detectors are used with different wavelength bandpass and/or neutral density filters, allowing the simultaneous detection of scatter at different wavelengths from different nanoparticles, or fluorescence light from the fluorescently coded microparticles, respectively.

In a dot plot, each particle is represented by a dot, positioned on the X and Y scales according to the intensities detected for that particle. Scatter dot plots (X = one laser scatter intensity; Y = second laser scatter intensity) are often informative (see examples below). Scatter scales can be either linear or log. Fluorescence dot plots typically plot X = one (e.g. green) fluorescence intensity, Y = another (e.g. red) fluorescence intensity. These two-color dot plots are often divided into four quadrants, the double negative cells, the green-only, red-only, and double positive cells. These are quantified by giving the percentage of cells in each quadrant. Since fluorescence intensity often varies several orders of magnitude between cells, the scales are usually the logarithm of fluorescence intensity spanning four logs (a 10,000-fold range).

Histograms are often used to interpret results of a flow-based assay. In a histogram, the X axis is intensity (of scatter or fluorescence), and the Y axis shows how many particles had each intensity. Thus, histograms show the distribution of intensities for a single parameter, while dot plots show the correlated distribution for two parameters. The density of dots in a region of a dot plot shows the "number of cells", equivalent to the Y axis of a histogram. Indeed, dot plots are sometimes represented as pseudo-3D graphs where the Z axis is "number of cells".

As shown in the Examples herein, it is feasible to detect scatter light from individual gold and silver nanoparticles using a standard flow cytometer, and distinguish between different sizes and types of nanoparticles. More importantly, changes in nanoparticle scatter induced by several different phenomena can also be detected and differentiated. Most notably, the change in light scatter of 60 nm Au complementary DNA binding-induced aggregation was sufficiently high to make these nanoparticle aggregates detectable and countable. Therefore, the aggregation of two nanoparticles which are brought together via binding by linker oligonucleotides to a target oligonucleotide can be detected using flow cytometry. Aggregated nanoparticles formed very bright and tight scatter profiles, making them easy to detect, differentiate from nanoparticle monomers, and quantify.

Numerous parameters make themselves amenable to nanoparticle detection, permitting those of skill in the art to design sufficiently discriminating gating strategies. With the right parameters and sufficient signal intensity, detecting and quantifying very rare events, even straight nanoparticle-protein/DNA-nanoparticle complexes, is feasible. Multiplexing of analytes can be performed by including other unique tags or even different sized nanoparticles in the complex. Furthermore, the real-time nature of flow cytometry makes it easier to break down the assay for better quality control of materials and detecting causes of non-specific binding.

An alternative way to analyze beads or tags is through capillary electrophoresis instead of flow cytometry. The concept is similar in that tags pass by an interrogation window in the capillary in single file, and are analyzed by laser-induced fluorescence measurement to decode the tags and quantify the captured target.

Another alternative is to visualize and analyze individual particles to measure changes in size by correlating with changes in speed in a solution and changes in brightness when illuminated.

In one embodiment of the present invention, a method is provided for detecting for the presence of one or more target analytes (or biobarcodes) in a sample, each target analyte having at least two binding sites for specific binding interactions with specific binding complements, in a sample.

In another embodiment of the present invention, several different target analytes (or biobarcodes) may be detected, where each target analyte has at least two binding sites for specific binding interactions with specific binding complements, in a sample.

In another embodiment of the present invention, several kinds of particle beads and several kinds of nanoparticle probes may be used to allow detection of multiple target analytes or biobarcodes. For instance, linkers that bind to a first kind of analyte would also bind to a particular size nanoparticle, and a particle bead with a particular fluorescent marker attached. Different combinations of nanoparticles and particle bead/fluorescent markers will allow for the detection of various different target analytes in the same solution.

Figure 6 provides an illustration of certain embodiments of the invention. Figure 6A depicts the detection of a target molecule, said target having at least a first portion and a second portion The detection of the target is accomplished using two linker oligos (linker A and linker B), a nanoparticle probe A, and a nanoparticle probe B. Nanoparticle probe A is conjugated with at least one oligonucleotide sequence A (in this case, d(AC)15). Linker A comprises at least a first portion and a second portion, said first portion comprising an oligonucleotide sequence A' (e.g., d(GT)15) complementary to oligonucleotide sequence A, and said second portion comprising a sequence complementary to the first portion of the target. Nanoparticle probe B is conjugated with at least one oligonucleotide sequence B (dT30). Linker B comprises a first portion and a second portion, said first portion comprising an oligonucleotide sequence B' (dA30) complementary to the oligonucleotide sequence B, and said second portion comprising a sequence complementary to the second portion of the target.

### Examples

The following examples are offered to illustrate, but not to limit, the invention.

### Example 1: Scatter light generated by gold and silver nanoparticles in flow cytometry assays

Gold and silver nanoparticles of various sizes were obtained from BBInternational Ltd. (Cardiff, UK) and used to demonstrate the capability of nanoparticles to be used in flow cytometry assays. Using a Cyan flow cytometer (Dako CytoMation, Ft. Collins, CO), 488 nm laser side scatter (SS Log) trigger intensity was adjusted to detect sub-micron particles. Side scatter light from 10⁶ 40nm or 60 nm gold or silver nanoparticles in 500 uL 4X SSC (Saline Sodium Citrate, Invitrogen) were measured in a 60 sec analysis (Figure 1B-D), and were compared to measurement of 4x SSC alone (Figure 1A). As shown in Figure 1, with respect to the size and material, counting efficiency for silver is better than gold particles and increased particle size results in increased detectability. Aggregated nanoparticles might be the cause of the counts seen away from the main population.

As shown in Figure 1 E-F, silver staining of gold nanoparticles causes a large shift in side scatter, indicating a significant change in particle size and scatter properties. Approximately 2x10⁸ 40 or 60 nm gold nanoparticles (in 2 uL stock buffer) were mixed with silver staining solution (2 uL Signal Enhancement A (SEA) + 2 uL Signal Enhancement B (SEB); both SEA and SEB are commercially available from Nanosphere, Northbrook, IL)) and reacted for 5 minutes at room temperature. Alternative functionally equivalent silver staining solutions are also available, e.g. Silver Enhancement Solution A, #S-5020 and Silver Enhancement Solution B, #S-5145 Sigma-Aldrich, St. Louis, MO. The reaction was stopped by diluting 2 uL with 600 uL 4x SSC. Scatter was detected with the Cyan 488 nm laser. Silver coatings on gold nanoparticles were found to significantly increase detectability of the gold nanoparticles. These results indicate that changes in side scatter intensity are sufficient to distinguish nanoparticles by substance, surface coating and size, which would be useful for multiplexing.

### Example 2: Detection and differentiation of nanoparticles using plasmon resonance-targeted wavelength light

Plasmon scatter light from silver particles can be seen by flow cytometry. A 100 uL aliquot of SEA was transferred to a clear 1.5 mL tube and exposed to ambient light. Using the CytoMation 405 nm laser and 430 nm filter, silver particles induced by exposure to light were detected by side scatter (Figure 2A(3) compared with Figure 2A(1) control). An increased 430 nm signal was also detected (Figure 2A(4) compared with Figure 2A(2)), indicating the peak plasmon scatter light from silver particles can be seen by flow cytometry using a plasmon resonance-targeted wavelength of light. Analyzing 30, 40, 50, 60 and 80 nm gold nanoparticles diluted to 16 fM in phosphate-buffered saline (PBS) run for 1 minute each on the Dako CytoMation MoFlo cell sorter by triggering on 488 nm signal and detection of 535 nm laser scatter allowed us to resolve differences in relative scatter between the particles based on plasmon resonance (Figure 2B). The relative level of detection was calculated by dividing the amount of particles detected after one minute by the expected amount of particles. Figure 2D was obtained by diluting 80 nm gold NP in 1 mL of water and counting 800 uL on a Partec CyFlow ML by triggering on 535 nm scatter and detecting 635 nm laser scatter. This demonstrates that individual nanoparticles can be detected and accurately counted above background due to plasmon resonance scatter.

To determine if results discussed above using flow cytometry were consistent with Mie's theory, the size-dependent scatter intensity of nanoparticles on a MoFlo cytometer was compared to the size-dependent change in intensity of the peak scatter wavelength when illuminated with white light from an HL-1 halogen source and detected using a USB2000 fiber optics spectrophotometer (Ocean Optics, Dunedin, FL). The intensity of the particle solutions analyzed on the MoFlo flow cytometer was normalized to levels over 30 nm intensity (set as 1) and plotted on a linear scale against the particle size. Figure 2E shows that the relative 535 nm laser light scatter intensity of various sizes of gold nanoparticles increases to the 6^{th} power of the particle diameter, similar to white light scatter, meaning that larger, more intense particles are more likely to be counted than smaller, less intense particles. See also Figure 2C. Larger particles can be counted with nearly 100% efficiency, as shown in Figure 2D. This demonstrates that the results conform to Mie's theory of particle light scatter.

### Example 3: Sorting of aggregated from monomeric nanoparticles

The scatter intensity of nanoparticle aggregates was also tested using the Ocean Optics spectrophotometer and compared to the intensity of aggregates measured by flow cytometry. 60 nm nanoparticle probes were aggregated using ioriic conditions under which the surface charge leads to particle aggregation (incubation in 0.8 M NaCl). Aggregated probes were concentrated by centrifugation at 200 x g for 15 min at RT. The scatter properties of singlet and aggregated nanoparticles were measured on an Ocean Optics spectrophotometer with white light illumination to show the shift in the resonance frequency. Particles and aggregates were then analyzed on a MoFlo flow cytometer (565 nm laser trigger and 635 nm detection wavelength) and sorted (fractionated) based on their scatter intensities (FL4 window). The sorted particle fractions were re-analyzed on the cytometer to check for purity and to establish that particle aggregates can be clearly identified and sorted by this method. As shown in Figure 3, the enriched aggregate sample had an increased right-angle white light scatter detected by the spectrophotometer (Figure 3A(2), compare with Figure 3A(1)) and an increased bright FL4 population detected by flow cytometry (Figure 3B(2), compare with Figure 3(B)(1)). The enriched aggregate sample was sorted based on FL4 intensity. Purity check showed they had sorted into two distinct populations (Figure 3B(3) and (4)).

### Example 4: Aggregation of nanoparticles detectable using flow cytometry

DNA-functionalized nanoparticles will self-assemble into aggregate structures that will have a higher plasmon resonance peak wavelength, which will scatter higher wavelength laser light with greater intensity than monomer particles. This is demonstrated by mixing probes functionalized with either poly-d(T) or poly-d(A) together (Figure 4A).

Various sizes of dT30 gold nanoparticles (Nanosphere, Northbrook, IL) were mixed with the same size dA30 gold nanoparticles (Nanosphere, Northbrook, IL) in 4X SSC at room temperature for 30-60 minutes. 1-2 uL of the mix were resuspended in 600 uL 4X SSC and analyzed with the Partec CyFlow ML flow cytometer (535 nm laser trigger, 635 nm laser detection). The complementary DNA-induced aggregation showed a time-dependent increase in a population with a higher 535 nm and 630 nm intensity (Figure 4B(2)-(4)), compared with the initial monomer population, seen as a low SSC red Log intensity peak (Figure 4B(1)).

Using 60 nm probes, 6 different concentrations of the d(T)30 probes were mixed with 100 fM of d(A)30 probe in 12 aliquots and assayed using a 96-well plate sampler. The ratio of aggregated probe over monomer probe was plotted vs. d(T)30 probe concentration, revealing a target-induced dose response (Figure 4C).

### Example 5A-B: Target analytes detected using nanoparticle probes and flow cytometry

This Example illustrates the detection of a target nucleic acid using nanoparticle probes and flow cytometry. 60nm gold nanoparticles functionalized with either poly-d(GT)₁₅ or poly-d(T)₃₀ oligonucleotides complementary to tails on a synthetic oligonucleotide target were used for this Example (Figure 5A). Target oligonucleotides in the concentrations indicated in Figure 5B were mixed with 5 pM each of the two complementary nanoparticle probes in 4x SSC and incubated at RT. After 10-30 min hybridization, a 1,000-fold dilution of the samples were analyzed by flow analysis (Figure 5B), trigger on 635 nm laser signal and detected with 535 nm laser. Increased target concentration increased the amount of aggregated gold NP, represented by the increase of the FL4 bright population. The amount of target detected was better than detection by white light side scatter. See Storhoff, J. J., A. D. Lucas, et al. (2004). "Homogeneous detection of unamplified genomic DNA sequences based on colorimetric scatter of gold nanoparticle probes." Nat Biotechnol 22(7): 883-7.

### Example 5C-D: Detection of target analytes using nanoparticle probes and Particle Tracking Analysis

Figure 5C illustrates how nanoparticles and aggregated nanoparticles can be identified and differentiated by a different process than flow cytometry, i.e., by the particle tracking analysis. See Carr, B, Diaper, T, and Barrett, E. NanoParticle Tracking Analysis - The Halo™ system. Royal Society of Chemistry Particle Systems Analysis Meeting. 2005 and Malloy, A and Carr, B. NanoParticle Tracking Analysis - The Halo™ System. Part Syst Charact 23(2), 197-204. 2006. The concept in principle is the same as the flow cytometry method described above, in that individual particles or aggregates are being measured in a homogeneous solution. During flow, these particles are temporarily isolated by physical confinement (i.e. when passing through the flow cell), and their signal can now be measured in the absence of the background generated by all the other particles. In the particle tracking analysis system, individual particles or aggregates are 'isolated' by image analysis; i.e. the diffraction limited signal is observed from individual particles and compared to their immediate surrounding. Then the speed (e.g. Brownian motion) with which these 'isolated' particles move is measured and used to determine their relative size. The sizes of the particles or aggregates were plotted on a histogram. This, in addition to intensity, allows differentiation of diffraction limited signals from monomeric particles and aggregates.

### Example 6: Viral target detected using nanoparticle probes and flow cytometry

As shown in Figure 6, detection of a viral target that has at least a first portion and a second portion, can be accomplished using nanoparticle probes. The detection of the target was accomplished using two linker oligos (linker A and linker B), a nanoparticle probe A, and a nanoparticle probe B (illustrated in Figure 6A). Nanoparticle probe A is conjugated with at least one poly d(AC) oligo. Linker oligo A comprises at least a first portion and a second portion, said first portion comprising a poly d(GT) oligo (complementary to the poly-d(AC) oligo conjugated to Nanoparticle probe A) and said second portion comprising a sequence complementary to the first portion of the viral target. Nanoparticle probe B is conjugated with at least one poly-dT oligos. Linker oligo B comprises a first portion and a second portion, said first portion comprising a poly dA oligo (complementary to the poly dT oligo conjugated to Nanoparticle probe B), and said second portion comprising a sequence complementary to the second portion of the viral target.

To detect the viral target derived from the West Nile Virus genome, a complex (as illustrated in Figure 6A) was formed by mixing an equimolar ratio of target oligo (5'-TGA CCA GTG CTA TCA ATC GGC GGA GCT CAA AAC AAA AGA AAA GAG GAG GAA AGA CCG GAA TTG CAG TCA TGA TTG-3' SEQ ID NO: 1) and linker oligonucleotides (Linker Probe A: 5'-d(GT)15 - CAA TCA TGA CTG CAA TTC CGG TCT TTC CTC CTC TT-3' SEQ ID NO: 2; Linker Probe B: 5-TTG AGC TCC GCC GAT TGA TAG CAC TGG TCA - d(A)30 SEQ ID NO: 3; all synthesized by IDT, Coralville, IA) in 4X SSC (20X SSC (Ambion, Austin, TX, cat#9770), diluted with DNA-grade water (Fisher Scientific, Pittsburgh, PA, cat# BP2470-1) for 5 minutes at 80°C. The mixture was cooled to room temperature and serially diluted 3-fold. Then, 10 pM total nanoparticle probe mix was added in 20 uL 4X SSC/4% dextran sulfate (Sigma-Aldrich, D-8906, MW 500,000; St. Louis, MO). The mixture was incubated at room temperature for 2 hours. Detection was performed by flow cytometry. 1 uL of the sample was mixed in 400 uL 4X SSC and scatter light intensity at 630 nm was measured (Figure 6B).

Another reaction mixture was formed by mixing 230 pM target complex in 8 pM of total nanoparticle probe mix in 20 uL 4X SSC/4% dextran sulfate. 1 uL of the sample was mixed in 400 uL 4X SSC and scatter light intensity at 635 nm was measured over time and the increase in dimer/monomer ratio was calculated (Figure 6C).

### Example 7: Detection of DNA target with 50 nm probes and protein target with 30 nm probes

A MecA assay was designed to detect Methicillin resistant Staphylococcus aureus (MRSA) with four 50 nm nanoparticle probes as illustrated in Figure 7A. 10 pM total nanoparticle probes produced as previously described (see Storhoff, J.J. and Lucas, A.D., Nat. Biotechnol 22(7): 883-7 (2004)) (2.5 pM each) in 70 uL 4X SSC/7.5% Formamide/4% dextran sulfate plus various concentrations of a 281 bp product derived by AmpliTaq PCR reaction from the MecA gene of MRSA (strain #700699 obtained from American Type Culture Collection, Manassas, VA) were incubated at room temperature for 1.25 hours. For flow cytometry, 1 uL of hybridization reaction was mixed with 1 mL 4X SSC, and a 565 nm laser was used for trigger and 635 nm laser to detect changes in scatter signal. The results shown in Figure 7B indicates a dose responsiveness between target concentration and the amount of aggregates.

### Example 8: Detection of protein by nanoparticle aggregation

To demonstrate that nanoparticle probes can be used to detect protein targets, 10 pM of 30 nm nanoparticles co-loaded with anti-PSA (prostate specific antigen) polyclonal antibody (R&D Systems, Minneapolis, MN) (and non-specific oligonucleotides added for use in biobarcode assays) were incubated with or without 200 ng prostate specific antigen (OEM Concepts, Toms River, NJ, Cat #H6M07-323) in PBS for 30 minutes at room temperature. An aliquot of each sample was diluted to 1 pM in PBS. The samples were run on the MoFlo flow cytometer for 1 minute, analyzed using a 565 nm signal and discriminated by pulse width. An increase in events brighter in FL3 and/or greater pulse width was detected, indicative of aggregated probe (Figure 8).

## Claims

1. A method for detecting for the presence of one or more target analytes in a sample comprising the steps of:
a) providing a plurality of nanoparticle probes conjugated to binding moieties capable of binding to a first binding site of the target analyte, wherein the nanoparticle probes comprise a metallic material and have an average diameter of less than 200 nanometers;
b) providing a capture surface comprising binding moieties capable of binding to a second binding site of the target analyte;
c) contacting the nanoparticle probes and capture surface with a sample believed to contain target analytes under conditions effective to allow for binding of the target analyte with the nanoparticle probes and the capture surface to form a complex in the presence of the target analyte;
d) optionally washing the capture surface containing the complex formed in (c) to remove non-bound nanoparticle probes;
e) releasing the captured nanoparticle probes from the capture surface;
f) subjecting the released nanoparticle probes to flow cytrometry or capillary electrophoresis, whereby individual nanoparticle probes can be detected;
g) illuminating nanoparticle probes in the confinement conditions of step f above with a light beam; and
h) measuring scatter light generated in step (g) to determine the number of released nanoparticle probes as an indicator of the presence of target analyte in the sample.

2. The method of claim 1, wherein the nanoparticle probes bind to the target analyte indirectly via specific linker molecules.

3. The method of claim 1, wherein the target analyte binds to the capture surface indirectly via at least one specific linker molecule.

4. The method of claim 1, wherein the capture surface is a microtiter well.

5. The method of claim 1, wherein the capture surface containing the complex formed in (c) is separated from non-bound nanoparticle probes.

6. The method of claim 5, wherein the capture surface is a magnetic bead.

7. The method of claim 1, wherein the plurality of nanoparticle probes comprises nanoparticle probes of different shapes, each differently shaped nanoparticle probe being conjugated to at least one binding moiety that binds to a different target analyte, and wherein each differently shaped nanoparticle probe creates unique scatter light when illuminated, thereby indicating the presence of the target analyte to which it binds.

8. The method of claim 1, wherein the plurality of nanoparticle probes comprises nanoparticle probes of different materials, each nanoparticle probe of different material being conjugated to at least one binding moiety that bind to a different target analyte, and wherein each nanoparticle probe of different material creates unique scatter light when illuminated, thereby indicating the presence of the target analyte to which it binds.

9. The method of claim 1, wherein the plurality of nanoparticle probes comprises nanoparticle probes of different sizes, each nanoparticle probe of different size being conjugated to at least one binding moiety that bind to a different target analyte, and wherein each nanoparticle probe of different size creates unique scatter light when illuminated, thereby indicating the presence of the target analyte to which it binds.

10. The method of claim 1, further comprising a step of providing one or more labeled microbeads that can bind to either the target analyte or to the nanoparticle probe, thereby capable of forming a complex with the nanoparticle probes and the target analyte.

11. The method of claim 10, wherein at least one microbead is fluorescently labeled.

## Patentansprüche

1. Ein Verfahren zum Nachweis des Vorliegens von einem oder mehr Zielanalyten in einer Probe, umfassend die Schritte:
a) Bereitstellen einer Vielzahl von Nanopartikelsonden, die an Bindungsgruppen konjugiert sind, die an eine erste Bindungsstelle des Zielanalyten binden können, wobei die Nanopartikelsonden ein metallisches Material umfassen und einen durchschnittlichen Durchmesser von weniger als 200 Nanometern haben;
b) Bereitstellen einer Einfangoberfläche umfassend Bindegruppen, die an eine zweite Bindungsstelle des Zienanalyten binden können;
c) Kontaktieren der Nanopartikelsonden und der Einfangoberfläche mit einer Probe, von der angenommen wird, dass sie Zielanalyten enthält, unter Bedingungen, die wirksam sind, das Binden des Zielanalyten an die Nanopartikelsonden und die Einfangoberfläche zu erlauben, um in der Gegenwart des Zielanalyten einen Komplex zu bilden;
d) optional Waschen der Einfangoberfläche, die den in (c) gebildeten Komplex enthält, um nicht gebundene Nanopartikelsonden zu entfernen;
e) Freisetzen der eingefangenen Nanopartikelsonden von der Einfangoberfläche;
f) Unterwerfen der freigesetzten Nanopartikelsonden unter Durchflusszytometrie oder Kapillarelektrophorese, wobei individuelle Nanopartikelsonden nachgewiesen werden können;
g) Beleuchten von Nanopartikelsonden unter den Einschränkungsbedingungen von obigem Schritt f mit einem Lichtstrahl; und
h) Messen von in Schritt (g) erzeugtem Streulicht, um die Anzahl der freigesetzten Nanopartikelsonden als einem Indikator für das Vorliegen von Zielanalyt in der Probe zu bestimmen.

2. Das Verfahren aus Anspruch 1, wobei die Nanopartikelsonden an den Zielanalyten indirekt über spezifische Linkermoleküle binden.

3. Das Verfahren aus Anspruch 1, wobei der Zielanalyt an die Einfangoberfläche indirekt über mindestens ein spezifisches Linkermolekül bindet.

4. Das Verfahren aus Anspruch 1, wobei die Einfangoberfläche ein Mikrotiternapf ist.

5. Das Verfahren aus Anspruch 1, wobei die Einfangoberfläche, die den in (c) gebildeten Komplex enthält, von nicht gebundenen Nanopartikelsonden getrennt wird.

6. Das Verfahren aus Anspruch 5, wobei die Einfangoberfläche eine magnetische Perle ist.

7. Das Verfahren aus Anspruch 1, wobei die Vielzahl von Nanopartikelsonden Nanopartikelsonden unterschiedlicher Form umfasst, wobei jede unterschiedlich geformte Nanopartikelsonde an mindestens eine Bindungsgruppe, die an einen unterschiedlichen Zielanalyten bindet, konjugiert ist, und wobei jede unterschiedlich geformte Nanopartikelsonde bei Beleuchtung einzigartiges Streulicht erzeugt, wodurch sie das Vorliegen des Zielanalyten, an den sie bindet, anzeigt.

8. Das Verfahren aus Anspruch 1, wobei die Vielzahl von Nanopartikelsonden Nanopartikelsonden aus unterschiedlichen Materialien umfasst, wobei jede Nanopartikelsonde aus unterschiedlichem Material an mindestens eine Bindungsgruppe, die an einen unterschiedlichen Zielanalyten bindet, konjugiert ist, und wobei jede Nanopartikelsonde aus unterschiedlichem Material bei Beleuchtung einzigartiges Streulicht erzeugt, wodurch sie das Vorliegen des Zielanalyten, an den sie bindet, anzeigt.

9. Das Verfahren aus Anspruch 1, wobei die Vielzahl von Nanopartikelsonden Nanopartikelsonden unterschiedlicher Größe umfasst, wobei jede Nanopartikelsonde unterschiedlicher Größe an mindestens eine Bindungsgruppe, die an einen unterschiedlichen Zielanalyten bindet, konjugiert ist, und wobei jede Nanopartikelsonde unterschiedlicher Größe bei Beleuchtung einzigartiges Streulicht erzeugt, wodurch sie das Vorliegen des Zielanalyten, an den sie bindet, anzeigt.

10. Das Verfahren aus Anspruch 1, weiter umfassend einen Schritt des Bereitstellens von einer oder mehr markierten Mikroperlen, die entweder an den Zielanalyten oder an die Nanopartikelsonde binden können, wodurch sie einen Komplex mit den Nanopartikelsonden und dem Zielanalyten bilden können.

11. Das Verfahren aus Anspruch 10, wobei mindestens eine Mikroperle fluoreszent markiert ist.

## Revendications

1. Procédé de détection de la présence d'un ou plusieurs analytes cibles dans un échantillon, comprenant les étapes consistant à :
a) fournir une pluralité de sondes nanoparticulaires conjuguées à des fragments de liaison capables de se lier à un premier site de liaison de l'analyte cible, les sondes nanoparticulaires comprenant un matériau métallique et ayant un diamètre moyen inférieur à 200 nanomètres ;
b) fournir une surface de capture comprenant des fragments de liaison capables de se lier à un deuxième site de liaison de l'analyte cible ;
c) mettre en contact les sondes nanoparticulaires et la surface de capture avec un échantillon censé contenir des analytes cibles dans des conditions efficaces pour permettre la liaison de l'analyte cible avec les sondes nanoparticulaires et la surface de capture pour former un complexe en présence de l'analyte cible ;
d) éventuellement, laver la surface de capture contenant le complexe formé à l'étape (c) pour éliminer les sondes nanoparticulaires non liées ;
e) détacher les sondes nanoparticulaires capturées de la surface de capture ;
f) soumettre les sondes nanoparticulaires détachées à une cytométrie de flux ou une électrophorèse capillaire, les sondes nanoparticulaires individuelles pouvant ainsi être détectées ;
g) éclairer les sondes nanoparticulaires dans les conditions de confinement de l'étape f ci-dessus avec un faisceau lumineux ; et
h) mesurer la lumière dispersée générée à l'étape (g) pour déterminer le nombre de sondes nanoparticulaires détachées comme indicateur de la présence de l'analyte cible dans l'échantillon.

2. Procédé selon la revendication 1, dans lequel les sondes nanoparticulaires se lient à l'analyte cible indirectement, via des molécules de liaison spécifiques.

3. Procédé selon la revendication 1, dans lequel l'analyte cible se lie à la surface de capture indirectement via au moins une molécule de liaison spécifique.

4. Procédé selon la revendication 1, dans lequel la surface de capture est un puits de microtitrage.

5. Procédé selon la revendication 1, dans lequel la surface de capture contenant le complexe formé à l'étape (c) est séparée des sondes nanoparticulaires non liées.

6. Procédé selon la revendication 5, dans lequel la surface de capture est une bille magnétique.

7. Procédé selon la revendication 1, dans lequel la pluralité de sondes nanoparticulaires comprend des sondes nanoparticulaires de formes différentes, chaque sonde nanoparticulaire de forme différente étant conjuguée à au moins un fragment de liaison qui se lie à un analyte cible différent et dans lequel chaque sonde nanoparticulaire de forme différente crée une lumière dispersée unique lorsqu'elle est éclairée, indiquant ainsi la présence de l'analyte cible auquel elle se lie.

8. Procédé selon la revendication 1, dans lequel la pluralité de sondes nanoparticulaires comprend des sondes nanoparticulaires de matériaux différents, chaque sonde nanoparticulaire de matériau différent étant conjuguée à au moins un fragment de liaison qui se lie à un analyte cible différent et dans lequel chaque sonde nanoparticulaire de matériau différent crée une lumière dispersée unique lorsqu'elle est éclairée, indiquant ainsi la présence de l'analyte cible auquel elle se lie.

9. Procédé selon la revendication 1, dans lequel la pluralité de sondes nanoparticulaires comprend des sondes nanoparticulaires de tailles différentes, chaque sonde nanoparticulaire de taille différente étant conjuguée à au moins un fragment de liaison qui se lie à un analyte cible différent et dans lequel chaque sonde nanoparticulaire de taille différente crée une lumière dispersée unique lorsqu'elle est éclairée, indiquant ainsi la présence de l'analyte cible auquel elle se lie.

10. Procédé selon la revendication 1, comprenant en outre une étape consistant à fournir une ou plusieurs microbilles marquées qui peuvent se lier à l'analyte cible ou à la sonde nanoparticulaire, pouvant ainsi former un complexe avec les sondes nanoparticulaires et l'analyte cible.

11. Procédé selon la revendication 10, dans lequel au moins une microbille est marquée par fluorescence.
